# EUROPEAN PATENT APPLICATION

(11) **EP 4 678 098 A1**
(43) Date of publication of application: **14.01.2026**
(21) Application number: 24187420.5
(22) Date of filing: 09.07.2024
(51) Int. Cl.: A61B 5/08, A61B 5/11, A61B 5/00, A61B 5/113, A61B 5/0205

(54) **DEVICE, SYSTEM AND METHOD FOR DETERMINING A WEARING STATUS OF A WEARABLE MOTION SENSOR TO BE WORN BY A USER**

(71) Applicant: Koninklijke Philips N.V., 5656 AG Eindhoven (NL)
(72) Inventor: HUIJBREGTS, Laurentia Johanna, Eindhoven (NL); COX, Lieke Gertruda Elisabeth, Eindhoven (NL); BONOMI, Alberto Giovanni, 5656AG Eindhoven (NL); RISPENS, Sietse Menno, Eindhoven (NL)
(74) Representative: Philips Intellectual Property & Standards

(57) **Abstract**

The present invention relates to a device, system and method for determining a wearing status of a wearable motion sensor to be worn by a user. The device comprises a sensor input configured to obtain a motion signal from the wearable motion sensor and a respiration signal from a respiration sensor; a processor configured to determine a correlation between the motion signal and the respiration signal for one or more time periods, and to determine a wearing status of the wearable motion sensor for the one or more time periods based on the determined correlation, and an output configured to output the wearing status.

## Description

### FIELD OF THE INVENTION

The present invention relates to a device, system and method for determining a wearing status of a wearable motion sensor to be worn by a user.

### BACKGROUND OF THE INVENTION

Studies suggest that monitoring the activity of users in hospitals, such as posture/turns, energy expenditure, or number of steps, gives relevant information e.g. to prevent pressure ulcers or determine discharge readiness.

Instead of putting an extra sensor on the user, it is preferred that the user's activity is monitored with a device that is already worn by the user. This makes a telemetry device a suitable candidate for activity monitoring. An accelerometer inside the device could be used to derive activity related metrics. The telemetry device at the same time may enable ECG- and SpO2-measurements and respiration rate could be determined from an impedance measurement over the ECG electrodes.

Although ECG cables and SpO2 sensors might still be on the body, the telemetry device might be taken off and for example be put on a nightstand or table or hung on an IV-pole. As a consequence, the device does not follow the motion of the user in the same way as when it was still worn and, for example, hanging around the neck. This could lead to missing events like turning or to misclassification of activities such as walking. For example, when the device is hung on an IV-pole and the user starts walking with the IV-pole, the device will not make the movements typical for walking for which a walking detection algorithm was designed (trained on users wearing the device). Therefore, in automatically generated reports about the user's activity, it might seem that he was sedentary the whole day, while in reality he/she walked a couple of times with the IV-pole. Therefore, it is important to know when the telemetry device is on and off body, respectively. In other words, in order not to draw wrong conclusions from the (absence of) (particular) motion of a monitoring device to be worn by the user, it should be recognized when the device is on or off the user's body.

WO 2017/190965 A1 discloses a method for verifying whether to change a determined wearing status of a device, the method comprising: acquiring a first sensor signal and a second sensor, both signals being indicative of whether a subject is wearing the device, determining a wearing status of the device based on the acquired first sensor signal, and whether to change the determined wearing status of the device based on the acquired second sensor signal, wherein when the wearing status indicates that the subject is not wearing the device and it is verified to change the determined wearing status, the method further comprises outputting a signal indicative that the subject requires assistance.

### SUMMARY OF THE INVENTION

It is an object of the present invention to provide a device, system and method for determining a wearing status of a wearable motion sensor to be worn by a user (particularly a patient). In particular, the wearing status shall be determined in a manner that the wearing status is reliable. Accordingly, it is an object to provide a reliable determination of a health state of a user if a wearable motion sensor and possibly other wearable user sensors are used to determine the health status of the user.

In a first aspect of the present invention a device for determining a wearing status of a wearable motion sensor to be worn by a user is presented, the device comprising:
- a sensor input configured to
   obtain a motion signal from the wearable motion sensor, and
   obtain a respiration signal from a respiration sensor;
   a processor configured to
   determine a correlation between the motion signal and the
   respiration signal for one or more time periods, and
   determine a wearing status of the wearable motion sensor for the one or more
   time periods based on the determined correlation, and
- an output configured to output the wearing status.

In a further aspect of the present invention a system for determining a wearing status of a wearable motion sensor to be worn by a user is presented, the system comprising:
- a device for determining a wearing status of a wearable motion sensor to be worn by a user, and
- the wearable motion sensor configured to detect motion of the user and to generate a corresponding motion signal.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea that a determination of a correlation of a motion signal from a wearable motion sensor with a respiration signal from the user (i.e. a determination whether a correlation of said signals is present or not) could help determine whether the user is wearing the wearable motion sensor or not. In other words, using the correlation between the motion signal and respiration signal, it can be determined whether the motion signal can be trusted, i.e. whether it indeed reflects motion of the user or whether the motion detected may be due to other causes.

Known solutions, as disclosed in WO 2017/190965 A1, for example, usually take into account a single sensor signal to determine the wearing status of a wearable sensor device and may use further sensor signals to verify whether the determined wearing status is correct or not. The signals used are independent of each other, however, and there is no plausibility check used for any one of said signals. For example, it is not checked whether motion, which appears to be due to respiration, really originates from respiration. Hence, in a case where a wearable user sensor, including a motion sensor, is hanging on an IV-pole moved by a user when walking, motion may be detected and thus it may be incorrectly concluded that the user is wearing the wearable user sensor. Accordingly, wrong conclusions about the user's health state may be drawn, particularly if the corresponding motion signal or another vital sign signal of the wearable device is used for further health analysis.

However, there are further reasons for which knowledge of the wearing status is important. Apart from the health state of the user it may be important to correctly interpret activity measurements. This aspect may particularly be important in view of training / fitness aspects. In hospitals or nursing homes, if a user does not wear a wearable device including a motion sensor, a warning signal that the device should be worn may be provided so that an automatic fall detection integrated into the device can be used. Furthermore, knowledge of the wearing status may improve power saving, i.e. if it is determined that the wearable motion sensor is not worn, the device and/or the wearable motion sensor may be put into a mode of low power consumption.

The respiration signal obtained does not necessarily have to be a signal from which respiration information, particularly a respiration rate or respiratory cycles, can directly be determined. In general, the respiration signals may be any vital sign signal from which respiration information can be determined in the end. Accordingly, the respiration signal may be one or more of a capnography signal, an impedance pneumography signal, a photoplethysmography (PPG) signal, electrocardiogram (ECG) signal, and a ventilator signal.

Hence, the respiration sensor does not necessarily have to be a respiration sensor in the "classical" sense, but may likewise include a ventilator. The respiration sensor may be any kind of device which provides a signal from which respiration information, such as a respiration rate or respirator cycle, can be determined (directly or indirectly). Thus, as a ventilator may provide respiration information based on how it steers, it may be regarded as respiration sensor in the context of the present invention.

The motion signal may comprise one or more of an accelerometer signal, a gyroscope signal, a magnetometer signal, and an air pressure sensor signal.

The processor is configured to determine a correlation between the motion signal and the respiration signal for one or more time periods (of said signals). In particular, the processor is configured to determine if there is a correlation between said signals in the sense that the respiratory information (e.g. respiratory rate and/or respiratory cycles), derived from the respiration signal can be seen back in the motion signal. In particular, the correlation between the motion signal and the respiration signal is determined for one or more of the "raw" signals, i.e. the signals as obtained, or said signals after processing (e.g. after filtering the signals, particularly after smoothing the signals, or after removing the DC component), the duration of one or more breathing cycles, the respiration rate (e.g. per minute).

However, it is also conceivable that the correlation provides information with respect to a more precise determination, particularly a degree of correlation, for example, whether there is a strong or weak correlation and possibly how strong or weak the correlation is. The degree of correlation may range between 0 and 100%, for example, wherein a 100% correlation implies full correlation, i.e. that variables used to quantify the correlation are exactly the same for both the motion signal and the respiration signal.

In general, the processor may be configured to determine the correlation by using spectral analysis, particularly coherence analysis and/or
wavelet transform analysis, and/or time analysis, particularly cross-correlation analysis
and/or dynamic time warping analysis, of the motion signal and the respiration signal.

In an embodiment of the device, the processor is configured to determine as the wearing status that the wearable motion sensor is worn if the correlation between the motion signal and the respiration signal is determined to be present. However, if no correlation is found it may not be determined that the wearable motion sensor is not worn. In fact, in absence of correlation, the wearable motion sensor may still be worn, for example in the cases of large motion, in particular wearing positions/arrangements, and also in case the respiratory signal does not have sufficient quality or is absent for some time.

In other words, in case a correlation is determined to be present or determined to be above a (predetermined) threshold, it may be concluded that the wearable motion sensor is worn by the user (particularly in touch with the user's body). In case it is determined that no correlation is present or is below a (predetermined) threshold, it may be concluded that the wearing status of the wearable motion sensor is "unknown" (or "off body").

However, the determined wearing status may not only indicate whether the wearable motion sensor is worn by the user (or not). In particular, if a degree of correlation is determined (and sufficiently graded), the wearing status may likewise indicate a probability for the user to wear the sensor (or not). In an embodiment of the device, the processor is further configured to determine if the motion signal is indicative of a predetermined motion of the user, wherein the processor is configured to determine as the wearing status that the wearable motion sensor is worn if the correlation between the motion signal and the respiration signal is determined to be present and/or if the motion signal is indicative of the predetermined motion.

Hence, to determine whether the wearable motion sensor is worn in a first step it may be determined whether a signal correlation is present and in a second step it may be determined if a motion corresponding to a pre-determined body movement (e.g. walking) is found in the motion signal and it may be determined that the wearable motion sensor is worn if at least one of the two is positive. Of course, the first step and the second step may be swapped. Besides, the second step may be skipped, i.e. if the motion signal is indicative of a pre-determined body movement it may be determined that the wearable motion sensor is worn (without determining a correlation between the motion signal and the respiration signal in a second step).

In another embodiment the processor is configured to adapt further processing of one or more parts of the motion signal (and possibly the respiration signal and/or another vital sign signal obtained), the one or more parts (portions) corresponding to the one or more time periods, based on the determined wearing status.

For example, if it is determined that there is no correlation between the motion signal and the respiration signal (or if the correlation is below a predetermined threshold) and hence it is determined that the wearable motion sensor is (most probably) not worn, assigning the motion signal to motions of the user would lead to wrong conclusions about the user's (health) state. Thus, it is important, that said motion signal is handled differently than a motion signal truly originating from a user's motion. The same applies to other signals measured by a wearable device comprising the motion sensor.

The processor may be configured to discard the one or more parts (portions) of the motion signal (and possibly the respiration signal and/or the other vital sign) signal based on the determined wearing status.

In particular, if the wearing status indicates that the user does not wear the wearable motion sensor the motion signal may be discarded. If the wearable motion sensor is part of a wearable device also measuring the respirations signal and/or another vital sign signal said signals may likewise be discarded in further processing, the reason being that these signals may not allow for correct conclusions about the user's health state.

In other words, the motion signal (and the respiration signal and/or another vital sign signal captured by a device comprising the wearable motion sensor) may be excluded from further processing if the determined wearing status indicates that the wearable motion sensor is not worn, i.e. on body.

The processor may be configured to tag the one or more parts (portions) of the motion signal, the respiration signal and/or the other vital sign signal based on the determined wearing status.

In particular, the processor may be configured to tag (i.e. mark) those parts of the motion signal, the respiration signal and/or the vital sign signal for which the determined wearing status indicates that the wearable motion sensor is worn. These parts may be tagged as "on body" or "worn". On the other hand, parts of the motion signal, the respiration signal and/or the vital sign signal for which the determined wearing status indicates that the wearable motion sensor is not worn may be tagged accordingly.

This way it can be easily derived whether said signal(s) may be used for further processing, such as assessing/determining patient activity tolerance, heart rate recovery after exercise etc., and/or drawing conclusions about a user's state or not. For example, activity tolerance, i.e. the user's ability to perform an activity or occupation without experiencing a disproportionate amount of physical, emotional, or physiological fatigue, may be determined by evaluating a change in vital signs in response to a predetermined activity such as walking a specific speed.

The processor may further be configured to select one or more algorithms for analyzing the one or more parts (portions) of the motion signal (and possibly the respiration signal and/or the other vital sign signal) based on the determined wearing status.

For example, for parts (i.e. portions) of said signal(s) for which it is determined that the wearable sensor is not worn, a different algorithm in further processing may be used as for those for which it is determined that the wearable motion sensor is worn. This way, algorithms tailored to unknown/off-body status and on-body status, respectively, may be used.

This way, activity-related metrics that one would like to derive from the motion signal of the wearable motion sensor, such as the user's activity level (i.e. how active is the user), the user's posture, when and for how long is the user walking, the user's walking speed, the number of steps of a user in certain period of time etc., may be determined in a reliable and precise manner.

When it is known that the wearable motion sensor is on the user's body and in which wearing position it is on the body (i.e. free with the cord around the neck / placed in right lower pocket of gown / placed in left upper pocket of gown /...), algorithms to derive activity-related metrics may be used that are tailored to this particular wearing position, resulting in very accurate results for the metrics to be determined.

In another embodiment of the device, the processor is configured to determine a position and/or orientation of the wearable motion sensor with respect to the user based on the motion signal if the determined wearing status indicates that the user is wearing the wearable motion sensor.

Using the wearing position and/or orientation with respect to the user for determining metrics from the motion signal and possibly the respirations signal and/or another vital sign signal measured taking into account the metrics determination may be improved.

In an embodiment of the device, the processor is configured to determine one or more of posture, activity type, activity amount and activity level from the motion signal if the determined wearing status indicates that the user is wearing the wearable motion sensor.

An activity type may be one or more of walking, climbing stairs, cycling, turning, swimming and so on. An activity amount may be a number of steps walked or climbed or a time span at or above a predetermine activity level or a time span in which the user performs a predetermined type of activity. An activity level may be represented by the speed of walking or cycling, , an intensity of the performed activity, and so on.

Determining the posture of the patient may particularly based on in information regarding the position and/or orientation of the wearable motion sensor with respect to the user (particularly as determined by the processor). However, said information may likewise be provided to the processor from an external source.

In case the activity level of the user is high, no correlation between the motion signal and the respiration signal may be found even when the user is wearing the motion sensor (particularly, for example, because a large motion of the user masks respiratory motion). However, to determine whether the user has worn the wearable motion sensor during said period of high activity (where the activity level is above a predetermined threshold) the processor may be configured to determine whether a correlation between the motion signal and the respiration signal is present in a period (directly) before and after said period of high activity. If a correlation can be found in the periods before and after the period of high activity, the processor may determine that the user has worn the wearable motion sensor also during the period of high activity.

In an embodiment of the device, the device may further comprise one or more of the wearable motion sensor, the respiration sensor, another vital sign sensor (configured to detect another vital sign of the user and to generate another vital sign signal), and a rendering unit (configured to issue visual and/or audible information in accordance with the determined wearing status, particularly to issue a warning signal if the wearing status indicates that the user is not wearing the wearable motion sensor).

The disclosed system comprises a device as described, and a wearable motion sensor configured to detect motion of the user and to generate a corresponding motion signal.

In an embodiment of the system, the system may further comprise one or more of
- a respiration sensor configured to detect respiration of the user and to generate a corresponding respiration signal,
- another vital sign sensor configured to detect another vital sign of the user and to generate another vital sign signal,
- a second wearable motion sensor configured to detect motion of the user and to generate a corresponding second motion signal, and
- a rendering unit configured to issue visual and/or audible information in accordance with the determined wearing status, particularly to issue a warning signal if the wearing status indicates that the user is not wearing the wearable motion sensor.

The wearable motion sensor, the respiration sensor, the other vital sign sensor, the second wearable motion sensor and/or the rendering unit may also be part of the device (and the device may be wearable).

The respiration sensor may be any type of sensor from which a respiration rate can be derived/determined (indirectly or directly). The respiration sensor may comprise one or more of an impedance sensor, a photoplethysmography (PPG) sensor, and a ventilator. Further, a capnography sensor, an ECG sensor, a respiratory inductance plethysmography (RIP) sensor, a vital signs camera, a pressure sensor in the mattress, or a pressure sensor in the nose may be used as respiration sensor.

Concerning the rendering unit, it is also conceivable that an advice/instruction is issued to the user and/or caregiver that the wearable motion sensor should be worn if the wearing status indicates that the wearable motion sensor is not worn by the user (for a time period exceeding a predetermined time period, for example).

The system may further comprise a device for further processing one or more parts of the motion signal and possibly the respiration signal and/or another vital sign signal, the one or more parts corresponding to the one or more time periods (for which a degree of correlation has been determined by the device for determining a wearing status), based on the determined wearing status. In particular, said device for further processing may be configured to discard and/or tag the one or more parts of the motion signal and possibly the respiration signal and/or the other vital sign signal based on the determined wearing status. Similarly, the device may be configured to select one or more algorithms for analyzing the one or more parts of the motion signal and possibly the respiration signal and/or the other vital sign signal based on the determined wearing status. Furthermore, said device for further processing may be configured to determine a position and/or orientation of the wearable motion sensor with respect to the user based on the motion signal if the determined wearing status indicates that the user is wearing the wearable motion sensor.

The other vital sign sensor may comprise one or more of an impedance sensor, a photoplethysmography (PPG) sensor, a ventilator, a capnography sensor, an ECG sensor, a respiratory inductance plethysmography (RIP) sensor, another motion sensor, a galvanic skin response sensor, and a pulse oximeter.

The second wearable motion sensor may particularly be configured to measure large body motions (instead of respiratory motion as the wearable motion sensor). The second wearable motion sensor may particularly be arranged in or on the same housing as the other components of the system. In particular, it may be arranged in/on the same housing as the wearable motion sensor. The second motion signal may be obtained by the processor for further processing said second motion signal, particularly for determining one or more of posture, activity type, activity amount and activity level.

Preferably, the system is configured to be worn by the user.

The term "worn" does not imply that the system has to be in direct touch with the user's body. In fact, skin contact is not required. Clothes, a bed sheet or other material could be located between the system, particularly the motion sensor of the system, and the user's body/skin. The system, particularly the motion sensor, may only loosely be attached to the user's body. For example, the system may be configured to be worn around the user's neck, in (a pocket of) a hospital gown of the user or any other body part allowing for (loose) attachment. Preferably, the system is a wearable that, when worn, is in (direct or indirect) touch with the torso, neck, and/or another portion of the user.

In an embodiment of the system, the wearable motion sensor comprises one or more of a wearable accelerometer, a wearable gyroscope, a wearable magnetometer, and a wearable air pressure sensor.

In an embodiment of the system, the motion sensor and the respiration sensor are configured to measure the motion signal and the respiration signal to be synchronized in time. In other words, the motion sensor and the respiration sensor may be configured to measure the motion signal and the respiration signal simultaneously (so that they are synchronized in time), i.e. in a synchronized manner. That is, the motion signal and the respiration signal may be synchronized in time.

In another embodiment of the system, the system is configured to adapt power consumption of one or more of the device, the wearable motion sensor, the respiration sensor, the other vital sign sensor and the rendering unit based on the determined wearing status, particularly to lower power consumption if the wearing status indicates that the user is not wearing the motion sensor.

The system may be configured to put said components individually or together into a power-saving mode if the determined wearing status does not indicate that the wearable motion sensor is worn.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention;
Fig. 2 shows a schematic diagram of an exemplary implementation of a system according to the present invention;
Fig. 3 shows a schematic diagram of a device according to the present invention;
Fig. 4 shows a diagram illustrating an exemplary use scenario of the system according to the present invention;
Fig. 5 shows a diagram of an activity level of a user over time;
Fig. 6 shows a flowchart of an embodiment of a method according to the present invention;
Fig. 7A sand 7B show a diagram illustrating a user wearing a wearable motion sensor of a system according to the present invention;
Fig. 7C shows the motion signals of the wearable motion sensor illustrated in Fig. 7A and 7B over a first period of time; and
Fig. 7D shows the motion signals of the wearable motion sensor illustrated in Fig. 7A and 7B over a second period over time; and
Fig. 7E shows the motion signals of the wearable motion sensor illustrated in Fig. 7A and 7B over a third period of time.

### DETAILED DESCRIPTION OF THE INVENTION

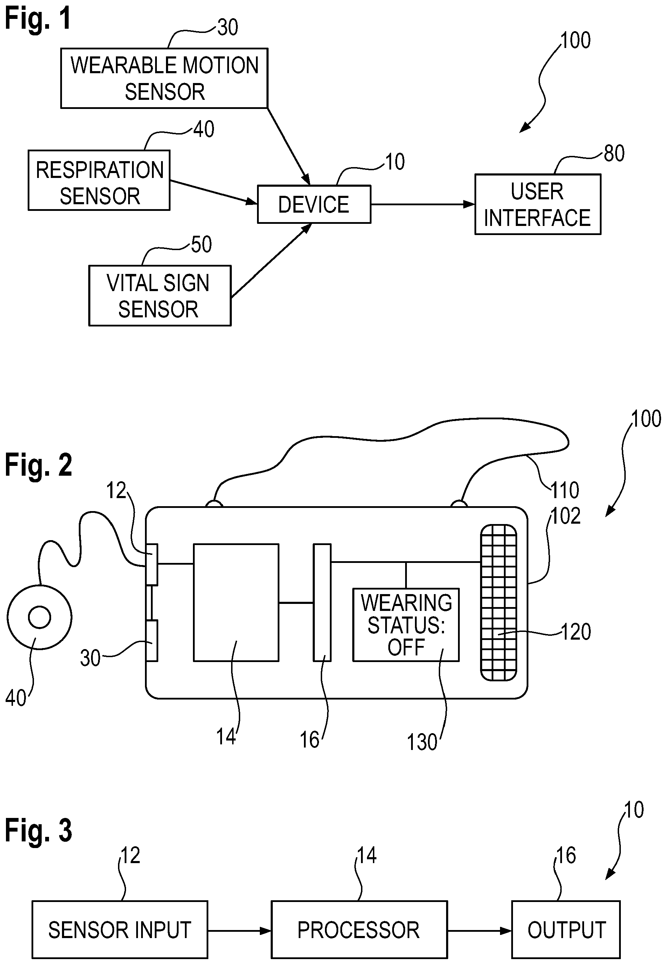

Fig. 1 shows a schematic diagram of an embodiment of a system 100 determining a wearing status of a wearable motion sensor to be worn by a user according to the present invention. The system 100 comprises the device 10 that is configured to determine a wearing status of a wearable motion sensor 30 to be worn by a user. The system 100 further comprises a wearable motion sensor 30 configured to detect (i.e. measure) motion of the user and to generate a corresponding motion signal.

The system may further comprise a respiration sensor 40 configured to detect (i.e. measure) respiration of the user and to generate a corresponding respiration signal, a vital sign sensor 50 configure to detect (i.e. measure) another vital sign of the user and to generate another vital sign signal and/or a rendering unit 80 configured to issue visual and/or audible information in accordance with the determined wearing status, particularly to issue a warning signal if the wearing status indicates that the user is not wearing the wearable motion sensor 30.

Preferably, the motion signal and the respiration signal are obtained in frequent and/or continuous manner.

In general, the respiration signal or the other vital sign signal may be obtained from other sources than the mentioned sensors. For example, the respiration rate could also be counted by a nurse or doctor and entered in the system via a user interface of the system 100. However, such counting is generally not preferred as it only provides infrequent data points and therefore does not allow for continuous and/or frequent checking of the correlation.

Similarly, information about another vital sign of the user could be obtained by a user interface of the system.

The wearable motion sensor 30 may be one or more of a wearable accelerometer, a wearable gyroscope, a wearable magnetometer, and a wearable air pressure sensor. The device 10 may obtain (i.e. receive or retrieve) a motion signal indicating motion of the user wearing the wearable motion sensor 30 from the wearable motion sensor. In general, the device 10 may derive/extract breath-by-breath information from the motion signal of the wearable motion sensor 30 if there is little noise (e.g. environmental noise, or noise due to other motion of the user) in said signal, while deriving a respiration rate (in particular from a power spectrum by Fourier analysis) might be the only possibility when there is more noise.

The respiration sensor 40 may comprise one or more of an impedance sensor, a photoplethysmography (PPG) sensor, a ventilator, a capnography sensor, an ECG sensor, and a respiratory inductance plethysmography (RIP) sensor. Some types of respiration sensors 40, such as impedance sensor, capnography sensor, or ventilator, provide respiration signals which are suited to derive (i.e. extract) breath-by-breath information (i.e. the duration of each respiratory cycle), while others (like photoplethysmography) are only suited to derive an (average) respiration rate Accordingly, depending on the type of respiration sensor 40 used, i.e. respiration signal obtained, the device 10 may only derive information of a respiration rate (instead of breath-by-breath information).

In case both the respiration signal and the motion signal comprise breath-by-breath information, the device 10 may compare the duration(s) of each respiratory cycle, respectively, to determine if there is a correlation between said signals. Additionally or alternatively, the correlation of the (raw) signals may be used (either before or after removal of noise factors).

In case the motion signal and/or the respiration signal only allow to determine (i.e. derive / extract) the respiration rate and not duration of each respiratory cycle, the power of the motion in the frequency band around the respiration rate (of the respiration signal) may be determined by the device 10. If this power is high and/or matches the expected power (or is above a predetermined power threshold), the processor may conclude as a wearing status that the wearable motion sensor 30 is worn. Of course, the power of the motion in the frequency band may also be determine in case breath-by-breath information (i.e. information of each respiratory cycle) is available.

The expected power (i.e. strength of the motion signal) can be estimated, using also the direction of the wearable motion sensor 30. When the direction of the wearable motion sensor 30 is such that it hints to lying on the back with the wearable motion sensor 30 on top of the user's body, the expected strength is larger than when the direction of the wearable motion sensor 30 is such that it hints to lying on the side, because for the former the motion of the torso is directly measured while for the latter the wearable motion sensor 30 might not touch the torso resulting in only indirect motion from movement of the mattress due to respiration.

Alternatively in case the motion signal and the respiration signal only allow to determine the respiration rate and not the duration of each respiratory cycle, the (raw) motion signal could be filtered such that frequencies lower and/or higher than (a predetermined threshold of) the respiration rate (as derived from respiration signal) are diminished. The remaining signal should then clearly show the respiration when the wearable motion sensor 30 is worn. Of course, the (raw) motion signal may likewise be filtered if breath-by-breath information is available.

The device 10 may be implemented in soft- and/or hardware, for instance by a programmed processor or computer. Device 10 and wearable motion sensor 30 (and optionally respiration sensor 40, vital sign sensor 50 and/or rendering unit 80) may be arranged in the same housing, i.e. integrated as a common device, e.g. a wearable device configured to be worn by the user. Alternatively, they may be implemented as separate entities, e.g. as a wearable motion sensor 30 worn by the user that transmits the measured motion signal, preferably in a wireless manner (e.g. via Bluetooth), to the device 10, where the measured motion signal is further processed, the result of which is provided to the rendering unit 80 for informing the user or another person, such a as a doctor or nurse of the user about the wearing status or related data. Similarly, the respiration sensor 40 may be arranged separately and the connection type to the device 10 may be adapted in accordance with the particular type of respiration sensor used. For example, a vital signs camera or a pressure sensor in the mattress may be arranged as remote sensors that solely have a wireless connection with the device 10. On the other hand, a pressure sensor in the nose may be arranged as a respiration sensor that has a wired connection with the device 10.

In general, different kinds of rendering unit 80 may be used in the system 100. Generally, one rendering unit may be sufficient, but multiple rendering units may be used as well. The rendering unit 80 may be any means that renders / outputs the estimated probability of the wearing status in visual or audible form, e.g. in text form, as image or diagram, as sound or spoken words, etc. For instance, the rendering unit may be a display, a loudspeaker, a touchscreen, a computer monitor, the screen of a smartphone or tablet, etc.

Fig. 2 shows a schematic diagram of an exemplary implementation of a system according to the present invention.

In this embodiment the system 100 comprises wearable motion sensor 30, device 10 (comprising sensor input 12, processor 14 and output16) display 130 and loudspeaker 120 are arranged in a single housing 102. In general, however, the display 130 and the loudspeaker 120 may be arranged in a remote manner and connected to the device 10 via a cable or wirelessly. The system 100 is a wearable system / wearable device in this embodiment. In particular, the system 100 is configured to be worn on or around the neck of the user. To this end, the system 100 comprises a cord 110 which is configured to be worn around the neck of the user. The system 100 further comprises a respiration sensor 40 which is located outside the housing 102. The respiration sensor 40 of this embodiment is connected to the sensor input 12 by a wire (particularly electrical wire). However, in general a wireless connection is likewise conceivable.

In other examples (not illustrated), the respiration sensor 40 may be arranged inside the housing. In some examples, the respiration sensor 40 may be positioned to be distant (i.e. remote or away) from the user.

The display 130 is a first rendering unit in the illustrated example, whereas the loudspeaker 120 is a second rendering unit. Both the display 130 and the loudspeaker 120 are connected to the output 16 of the device 10 and configured to obtain the wearing status from the output 16 and to render information in accordance with said wearing status.

Fig. 3 shows a schematic diagram of a device 10 according to the present invention. The device 10 generally comprises a sensor input 12, a processor 14 and an output 16. The device 10 may e.g. be implemented as or in a processing unit or computer, in software and/or hardware.

The sensor input of the device 12 may be configured to obtain a motion signal directly from a wearable motion sensor 30, configured to measure motion (particularly respiratory motion) of the user and to generate a corresponding motion signal, and a respiration signal directly from a respiration sensor 40, configured to detect respiration of the user and to generate a corresponding respiration signal. Thus, the sensor input 12 may be directly coupled or connected to said sensors. However, the sensor input 12 may likewise obtain one or both of said signals indirectly and thus may be directly coupled or connected to any one of a user interface configured to obtain the motion signal and/or the respiration signal or to derive said signals from other data, a database comprising the motion signal and the respiration signal, or may obtain (i.e. retrieve or receive) these data from a storage, buffer, network, or bus, etc. The sensor input 12 may e.g. be a (wired or wireless) communication interface or data interface, such as a Bluetooth interface, Wi-Fi interface, LAN interface, HDMI interface, direct cable connect, or any other suitable interface allowing signal transfer to the device 10.

The device 10 further comprises a processor 14 configured to determine a degree of correlation between the motion signal and the respiration signal for one or more time periods and to determine a wearing status of the wearable motion sensor 30 for the one or more time periods based on said degree of correlation. The processor 14 may be any kind of means configured to process the signals obtained and to determine the degree of correlation (and the wearing status) therefrom. It may be implemented in software and/or hardware, e.g. as a programmed processor or computer or app on a user device such as a smartphone, smartwatch, tablet, laptop, PC, workstation, etc.

The output 16 of the device 10 is configured to provide, particularly output, wearing status, particularly to a rendering unit 80 configured to render audible and/or visual information in accordance with the determined wearing status. The output unit 16 may generally be any interface that provides determined wearing status, e.g. transmits it to another device or provides it for retrieval by another device (e.g. a smartphone, computer, tablet, etc.). It may thus generally be any (wired or wireless) communication or data interface.

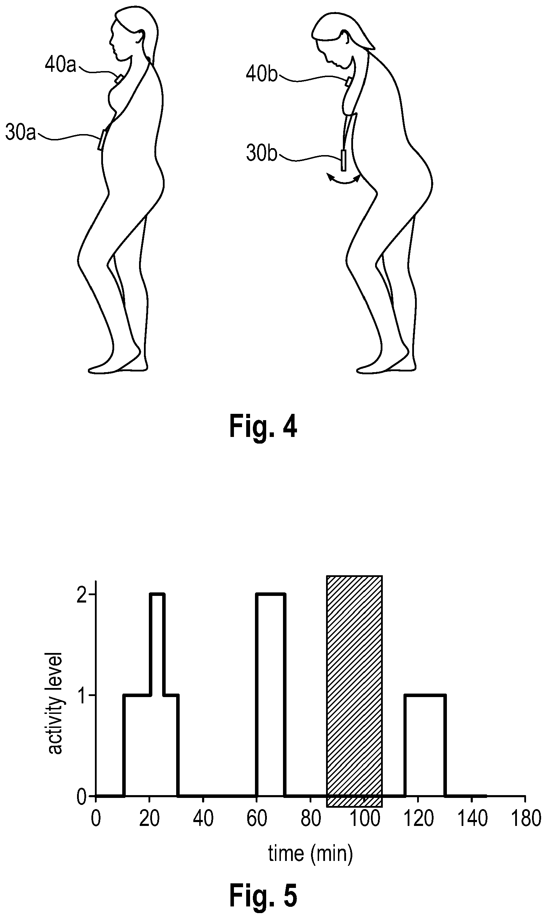

Fig. 4 shows a diagram illustrating an exemplary use scenario of the system 100 according to the present invention. The system 100 comprises two wearable motion sensors 30a and 30b, two wearable respiration sensors 40a and 40b, a device 10 and a rendering unit 80.

For the user shown on the left side, both the wearable motion sensor 30 and the respiration sensor 40 are in contact with the user's body. For the user on the right side, the wearable motion sensor 30b fails to be in contact with the user's body. Rather, the wearable motion sensor 30b, which is a pendant worn by the user around the neck, is displaced from the user's body and swings with every step of the user, which is due to the user leaning forward.

As to the left user, the device 10, configured to obtain a motion signal from the wearable motion sensor 30a and a respiration signal from the wearable respiration sensor 40a (e.g. in a wireless manner), will find a (high degree of) correlation between the motion signal and the respiration signal and will thus determine as the wearing status that the wearable motion sensor 30a is worn. This is due to the fact that both sensors 30a and 40a are closely worn to the user's body. Accordingly, the wearable motion sensor 30a is able to sense respiratory motion of the user. In general, it can be expected that the device 10 is configured to find a (high degree of) correlation when the user is sitting, lying, or standing upright (in a still manner). On the other hand, when the user is moving (turning, walking, etc.) or leaning forward as the user on the right it may be more challenging for the device 10 to find a correlation between respiration signal and the motion signal, which is due to the fact that respiratory motion cannot be detected, because the wearable motion sensor 30 is not close enough to the user's body, or is superimposed by other user motions. However, if right before and right after a motion, respiratory motion is detected, it may be assumed that during the motion the wearable motion sensor was worn.

Similarly, although not shown, when the device is hung on an IV-pole, while pushed by the user during walking, or when the user is walking with a bent posture as shown on the right of Fig. 4, no respiratory motion will be detected by the wearable motion sensor. However, in the processor (not shown) of the device 10 may be configured to select particular algorithms for processing the motion signal (based on the correlation determined to be low / non-existent) to determine whether the obtained motion signal is indicative for walking with an IV-pole, or walking with bent posture, respectively. In fact, dedicated algorithms, tailored to either IV-pole walking or walking with bent posture may be used by the processor to determine activity-related metrics like walking speed and number of steps. On the other hand, when respiratory motion is detected and thus it is found that a (high) correlation with the respiration signal is existent, on-body algorithms may be used to determine activity-related metrics like activity level, whether the user is walking, and if yes, for how long, with which speed, and how many steps are taken etc. In case bent walking is detected, a warning could be given to the user or nurse/physiotherapist that the user should try to walk more upright, as this is usually better for the body.

Fig. 5 shows a diagram of an activity level (activity signal) of a user over time. Such a diagram could be rendered by the rendering unit 80 of the system 100 according to the present invention.

The activity signal is a signal derived from the motion signal here in this case. As can be seen from the diagram periods of higher activity alternate with periods of less or even zero activity. However, as already mentioned, such data might not reflect a user's true activity level if it is not ensured that the underlying motion signal originates from a wearable motion sensor that is indeed worn. In other words, if is determined that the motion signal of the user is not correlated with his/her respiration signal, i.e. if it is determined for some periods of said signals that the wearable motion sensor is not worn, the motion signal - and hence the derived activity signal - for said periods may not be trustworthy. In the diagram shown this is the case for the time period marked with the hatched block. In this period the wearable motion sensor is has been determined by the processor 14 of the system to not be worn. Due to the marking/tagging of the data by the processor 14 a clinician who views the data thus knows which part of the data he/she cannot trust to draw conclusions. In this period, where the wearing status of the device is negative (i.e. where the device is not worn), the activity level has been determined to be zero. However, this does not necessarily imply that the user was not active. In fact, the device might have been lying on a table, while the user was active, for example.

Alternatively or additionally, to marking /tagging those periods of the activity signal, the corresponding parts of said signal may not be used for further analysis (by the processor or a caregiver, doctor etc.). Accordingly, different and particularly more trustworthy conclusions about the health state of the user may be drawn. For example, for the diagram of Fig. 5, not neglecting the activity signal part in the grey block, the following could be concluded:
activity level 0: 100 min, 69 % of the time
activity level 1: 30 min, 21 % of the time
activity level 2: 15 min, 10 % of the time.

However, if said part of the signal was neglected in further processing / analysis and particularly neglected in determining the health state of the user, the total time of useful motion signal data would not amount to 145 minutes, but only to 125. Accordingly, the following could be concluded:
activity level 0: 80 min, 64 % of the time
activity level 1: 30 min, 24 % of the time
activity level 2: 15 min, 12 % of the time
(unknown: 20 min).

In the example of Fig. 5, the raw accelerometer data, i.e. the motion signal, in the period where the device was not worn were anyway processed to determine an activity level (it resulted in activity level 0). Another option would be not to process the motion signa at all once the wearing status has been determined. Therefore, when it has been determined that the device is not worn in a certain period of time, (for this example) the activity level will not be determined at all. In other example, in an off-body time period no classification may take place as to whether the user is walking or not and no algorithm may run to count steps. Accordingly, the device may save power for said time period.

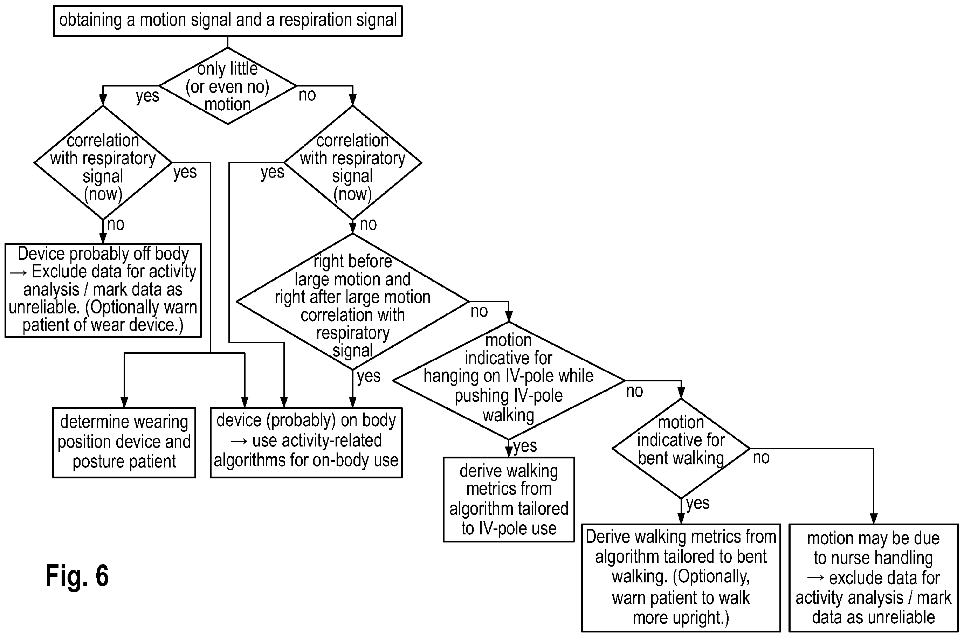

Fig. 6 shows a flowchart of an embodiment of a method according to the present invention.

In a first step of the method a motion signal (from a wearable motion sensor) and a respiration signal (from a respiration sensor) are obtained. Before determining a (existence of) correlation between the motion signal and the respiration signal for one or more time periods, it may be determined whether the motion signal reveals only little or even no (respiratory) motion of the user in said time periods. In case only little or no motion is detected and furthermore there is no correlation found between the motion signal and the respiration signal, the wearing status of the wearable motion sensor may be determined to be negative, i.e. it may be determined that the wearable motion sensor is not worn during said time period(s).

On the other hand, if it is determined that there is a correlation between the signals, it may be determined that the wearable motion sensor is worn.

In a next step, the wearing position (and/or orientation) of the wearable motion sensor and thus the user could be determined from the signals obtained. Alternatively, based on the determined wearing status, i.e. that the wearable motion sensor is (probably) worn, algorithms suited for the on-body situation may be used to further process/analyze the sensor signals.

In case the motion is not determined to be little or even non-existent, and a correlation is found, the same steps follow as in the previous case.

However, if in this case it is determined that there is no correlation, the motion signal may further be analyzed using algorithms for off-body motion. For example, it may be determined whether the motion signal is indicative of a wearable motion sensor hanging on an IV-pole and being pushed during walking. If the motion signal is indicative of such motion, walking metrics of the user could be determined from the motion signal. If the motion signal is not indicative of such motion, however, it may be determined whether the motions signal is indicative of bent walking, for example. Similarly, if this is the case, walking metrics of the user could be determined. If not, the motion signal and the respiration signal may be excluded from further processing and/or marked as unreliable.

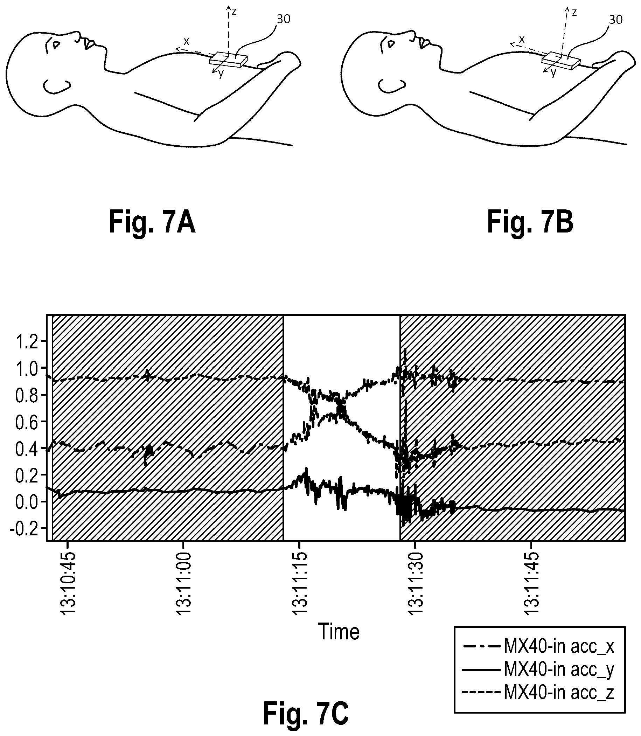

Figs. 7A and 7B show a diagram illustrating a user wearing a wearable motion sensor 30 of a system 100 according to the present invention. The wearable motion sensor 30 is particularly configured to measure the respiratory motion of the user. To this end, the wearable motion sensor is worn as a pendant around the user's neck (not shown) and resting on the user's thorax. However, the wearable motion sensor 30 could be worn elsewhere, for example, on the user's breast. Further, in this embodiment of the system 100, the wearable motion sensor 30 is an accelerometer configured to measure the motion of the user's thorax (which is mainly caused by respiration in the shown position of the user) in three directions, which are orthogonal to each other, i.e. the x-, y-, and z-direction as illustrated in Figs. 7A and 7B. Hence, the wearable motion sensor 30 is configured to provide three motion signals: an x-direction motion signal indicating a motion of the user's thorax in the x direction (pointing approximately to the head of the user), a y-direction motion signal indicating a motion of the user's thorax in the y direction (pointing to the right side of the user), and a z-direction motion signal indicating a motion of the user's thorax in the z direction (pointing approximately to the ceiling over the user). In general, when the wearable motion sensor 30 is worn, respiratory motion should be visible in at least one of said three directions. When lying on the back, both the center of gravity and the direction of the device should change due to the respiratory motion of the user. As can be seen from a comparison of Figs. 7A and 7B the vectors in the x- and z-direction are oriented slightly differently during expiration. The wearable motion sensor 30 is also moving a bit up (breathing in) and down (breathing out) as a whole.

Due to different points in time of a respiration cycle, the motion vectors in the three directions shown in Figs. 7A and 7B are slightly different. This effect becomes stronger, if the user's posture as captured in the figures was significantly different, for example, if the user shown in Fig. 7A was lying on his/her back while he/she was lying on his/her side in Fig. 7B.

Fig. 7C shows the three motion signals of the wearable motion sensor 30, illustrated exemplary in Fig. 7A and 7B, over a first period of time. During time period 13: 10:43 until 13:11:12 the user is lying on the back with the device resting on him/her as illustrated in Fig. 7A and 7B. The movement due to respiration can thus predominantly be seen in the x-direction (dotted line). After a transition to sitting by increasing the bed angle, the user is sitting from 13:11:28 until 13:11:55 (not shown). For this time period the motion signal indicates that there is respiratory motion predominantly in the z-direction (dashed line)

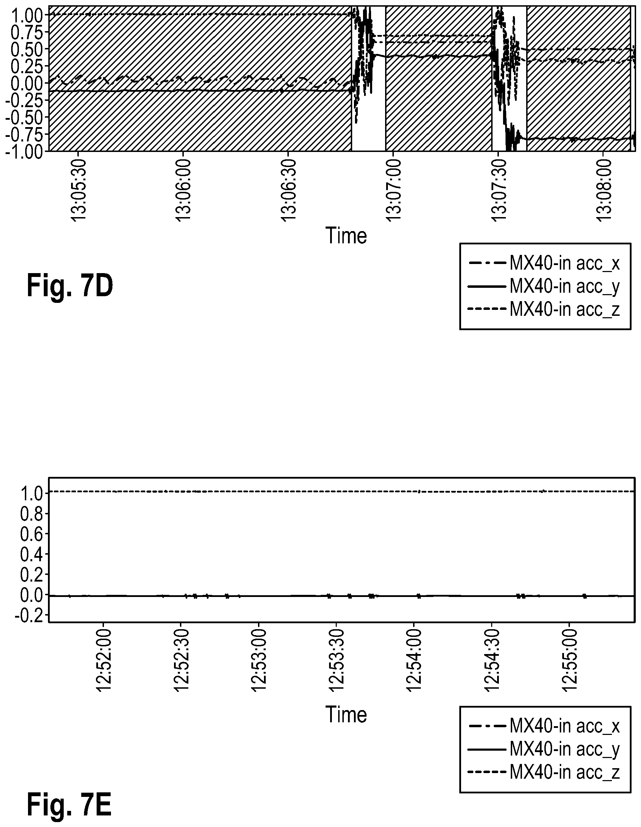

Fig. 7D shows the three motion signals of the wearable motion sensor 30 illustrated in Fig. 7A and 7B over a second period over time. In this period, the user is lying on the back until 13:06:47. Like in the diagram shown in Fig. 7C, motion due to respiration can then best be seen in the motion signal corresponding to the x-direction (dotted line). The user turns to the left side and is lying on her left side from 13:06:57 until 13:07:28, with respiratory motion predominantly being visible in the motion signal corresponding the y-direction (solid line). Afterwards the user turns on his/her right side and lies on his/her right side from 13:07:37 until 13:08:08 with respiratory motion predominantly being visible in the motion signal corresponding to the z-direction motion (dashed line).

When lying on the side, the wearable motion sensor 30 generally rests on the mattress (not shown). However, as the wearable motion sensor 30 might still touch a part of the body and as the mattress is also moving due to respiration, still respiratory motion is visible in the illustrated motion signals, although a bit weaker than when lying on the back (see middle and right part of the diagram of Fig. 7D in comparison to the left part of said diagram).

Fig. 7E shows the motion signals of the wearable motion sensor 30 illustrated in Figs. 7A and 7B over a third period of time in which the wearable motion sensor 30 is not worn by the user but lies on a table. In particular, the motion signals corresponding to motion in the y- and z-direction are shown. As can be seen, there is no respiratory motion visible from the signals shown.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (10) for determining a wearing status of a wearable motion sensor (30) to be worn by a user, the device comprising
- a sensor input (12) configured to
obtain a motion signal from the wearable motion sensor (30), and
obtain a respiration signal from a respiration sensor (40);
a processor (14) configured to
determine a correlation between the motion signal and the
respiration signal for one or more time periods, and
determine a wearing status of the wearable motion sensor (30) for the one or more time periods based on the determined correlation, and
an output (16) configured to output the wearing status.

2. Device (10) according to claim 1, wherein the processor (14) is configured to
determine as the wearing status that the wearable motion sensor (30) is worn if the correlation between the motion signal and the respiration signal is determined to be present.

3. Device (10) as claimed in claim 1, wherein the processor (14) is further configured to determine if the motion signal is indicative of a predetermined motion of the user, wherein the processor (14) is configured to determine as the wearing status that the wearable motion sensor (30) is worn if the correlation between the motion signal and the respiration signal is determined to be present and/or if the motion signal is indicative of the predetermined motion.

4. Device (10) according to anyone of the preceding claims, wherein the processor (14) is configured to adapt further processing of one or more parts of the motion signal and possibly the respiration signal and/or another vital sign signal obtained, the one or more parts corresponding to the one or more time periods, based on the determined wearing status.

5. Device (10) according to claim 3, wherein the processor (14) is configured to discard and/or tag the one or more parts of the motion signal and possibly the respiration signal and/or the other vital sign signal based on the determined wearing status.

6. Device (10) according to claim 3 or 4, wherein the processor (14) is configured to select one or more algorithms for analyzing the one or more parts of the motion signal and possibly the respiration signal and/or the other vital sign signal based on the determined wearing status.

7. Device (10) according to any one of the preceding claims, wherein the processor (14) is configured to determine a position and/or orientation of the wearable motion sensor (30) with respect to the user based on the motion signal if the determined wearing status indicates that the user is wearing the wearable motion sensor (30).

8. Device (10) according to any one of the preceding claims, wherein the processor (14) is configured to determine one or more of posture, activity type, activity amount and activity level from the motion signal if the determined wearing status indicates that the user is wearing the wearable motion sensor (30).

9. System (100) for determining a wearing status of a wearable motion sensor (30) to be worn by a user, the system (100) comprising,
- the device (10) according to claim 1, and
- the wearable motion sensor (30) configured to detect motion of the user and to generate a corresponding motion signal.

10. The system (100) according to claim 9, further comprising one or more of
- a respiration sensor (40) configured to detect respiration of the user and to generate a corresponding respiration signal,
- another vital sign sensor (50) configured to detect another vital sign of the user and to generate another vital sign signal,
- a second wearable motion sensor configured to detect motion of the user and to generate a corresponding second motion signal, and
- a rendering unit (80) configured to issue visual and/or audible information in accordance with the determined wearing status, particularly to issue a warning signal if the wearing status indicates that the user is not wearing the wearable motion sensor (30), and/or
wherein the system (100) is configured to be worn by the user.

11. System (100) according to claim 10,
wherein the wearable motion sensor (30) comprises one or more of a wearable accelerometer, a wearable gyroscope, a wearable magnetometer, and a wearable air pressure sensor.

12. System (100) according to any one of claims 9 to 11, wherein the wearable motion sensor (30) and the respiration sensor (40) are configured to measure the motion signal and the respiration signal to be synchronized in time.

13. System (100) according to any one of claims 9 to 12,
wherein the system (100) is configured to adapt power consumption of one or more of the device (10), the wearable motion sensor (30), the respiration sensor (40), the other vital sign sensor (50) and the rendering unit (80) based on the determined wearing status, particularly to lower power consumption if the wearing status indicates that the user is not wearing the wearable motion sensor (30).

14. Method for determining a wearing status of a wearable motion sensor to be worn by a user, the method comprising
- obtaining a motion signal from the wearable motion sensor,
- obtaining a respiration signal from a respiration sensor;
determining a correlation between the motion signal and the
respiration signal for one or more time periods,
- determining a wearing status of the wearable motion sensor for the one or more time periods based on the determined correlation, and
outputting the wearing status.

15. Computer program comprising program code means for causing a computer to carry out the steps of the method as claimed in claim 14 when said computer program is carried out on the computer.
